# EUROPEAN PATENT APPLICATION

(11) **EP 1 504 755 A1**
(43) Date of publication of application: **09.02.2005**
(21) Application number: 03721101.8
(22) Date of filing: 13.05.2003
(51) Int. Cl.: A61K 9/08, A61K 9/19, A61K 31/40, A61K 31/404, A61K 31/445

(54) **LYOPHILIZATION PRODUCT**

(30) Priority: 13.05.2002 JP 2002136881
(71) Applicant: DAIICHI PHARMACEUTICAL CO., LTD., Chuo-ku, Tokyo 103-8234 (JP)
(72) Inventor: FUJII, Yoshimine Daiichi Pharmaceutical Co.,Ltd., Edogawa-ku, Tokyo 134-8630 (JP); SUZUKI, Norio Daiichi Pharmaceutical Co., Ltd., Edogawa-ku, Tokyo 134-8630 (JP)
(74) Representative: Hartz, Nikolai F., Dr.
(86) International application number: PCT/JP2003/005940
(87) International publication number: WO 2003/094889

(57) **Abstract**

The present invention relates to an aqueous solution containing a physiologically active substance which may have a substituent and which has an amidino group, wherein the pH of the solution is higher than 2 and equal to or lower than 4. The invention also relates to a lyophilized product obtained by drying the solution, to an injection containing the aqueous solution or the lyophilized product, and to an injection kit.

## Description

### Technical Field

The present invention relates to an aqueous solution in which a physiologically active substance having an amidino group and an optional substituent can remain stable and which can be stored for a long period of time. The invention also relates to a lyophilized product of the solution, and to an injection containing the aqueous solution or the lyophilized product.

### Background Art

A variety of known physiologically active substances have an amidino group and an optional substituent. These substances having an amidino group and an optional substituent in the molecule thereof is unstable with the elapse of time (days). Thus, there is demand for a technique for maintaining these substances stable and storing them for a long period of time.

Meanwhile, it has been known that dry cake is difficult to obtain by lyophilizing a solution in which only a physiologically active substance, solid matter, is dissolved. Even though dry cake is not formed, the activity (pharmacological effect) of the physiologically active substance can be developed without any problem. However, in general, the presence of the content in a drug container such as a vial or an ample is visually confirmed upon dissolution of the content, and when no dry cake is observed in the container, whether or not a physiologically active substance is actually contained can be confirmed. In some cases, such a drug product is regarded a defective product, let alone exhibits poor appearance. Dry cake, which is generally amorphous and porous, is rapidly dissolved in a solvent which is employed prior to use. Therefore, formation of dry cake is an important issue for such lyophilized products.

There has been reported a lyophilized drug preparation containing a disaccharide for improving day-elapsing stability of a compound having an amidino group in its molecule (Japanese Patent Application Laid-Open (*kokai*) No. 9-71533).

### Disclosure of the Invention

The present inventors have carried out extensive studies on technical improvement in long-term storage of a physiologically active substance having an amidino group, and surprisingly, have found that the aforementioned problems relating to stability, formation of dry cake, etc. can be solved by adjusting the pH of an aqueous solution containing a physiologically active substance which may have a substituent and which has an amidino group to a value higher than 2 and equal to or lower than 4. The present invention has been accomplished on the basis of this finding.

Accordingly, the present invention provides an aqueous solution containing a physiologically active substance which may have a substituent and which has an amidino group and having a pH higher than 2 and equal to or lower than 4; an aqueous solution containing a physiologically active substance which may have a substituent and which has an amidino group and having a pH higher than 2 and lower than 4; an aqueous solution containing a physiologically active substance which may have a substituent and which has an amidino group and having a pH higher than 2 and equal to or lower than 3.5; and an aqueous solution containing a physiologically active substance which may have a substituent and which has an amidino group and having a pH of 2.5 to 3.5.

The present invention also provides a lyophilized product obtained by lyophilizing an aqueous solution containing a physiologically active substance which may have a substituent and which has an amidino group and having a pH higher than 2 and equal to or lower than 4; a lyophilized product obtained by lyophilizing an aqueous solution containing a.physiologically active substance which may have a substituent and which has an amidino group and having a pH higher than 2 and lower than 4; a lyophilized product obtained by lyophilizing an aqueous solution containing a physiologically active substance which may have a substituent and which has an amidino group and having a pH higher than 2 and equal to or lower than 3.5; and a lyophilized product obtained by lyophilizing an aqueous solution containing a physiologically active substance which may have a substituent and which has an amidino group and having a pH of 2.5 to 3.5.

The present invention also provides an injection comprising any of the aqueous solutions or any of the lyophilized products, and an injection kit comprising a solvent for reconstitution prior to use and the injection.

### Best Mode for Carrying out the Invention

In the present invention, the term "physiologically active substance which may have a substituent and which has an amidino group" refers to a physiologically active substance which may have in its molecule at least one substituent and which has an amidino group. No particular limitation is imposed on the species of the physiologically active substance. The amidino group may have a substituent, and examples of the substituent include C1 to C6 linear, branched, and cyclic alkyl groups. Specific examples of the alkyl groups include methyl, ethyl, isopropyl, and isobutyl.

Examples of the physiologically active substance of the present invention which may have a substituent and which has an amidino group include a compound represented by the following formula (1), a salt thereof, and a solvate of the compound or salt: 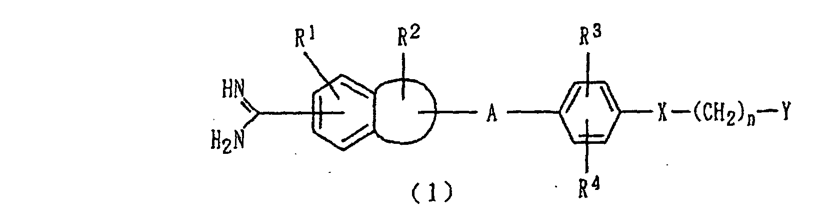 (wherein R¹ represents a hydrogen atom or a lower alkoxy group;
R² represents a hydrogen atom, a lower alkyl group, a lower alkoxy group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxy group, or an alkoxycarbonylalkoxy group;
R⁴ represents a hydrogen atom, a halogen atom, an amino group, a cyano group, a nitro group, a hydroxyl group, a lower alkyl group, or a lower alkoxy group;
n is a number from 0 to 4;
A represents a C1 to C4 alkylene group or group -B-N(R⁵)-, which may have 1 to 2 substituents selected from among a hydroxyalkyl group, a carboxyl group, an alkoxycarbonyl group, a carboxyalkyl group, and an alkoxycarbonylalkyl group (wherein B represents a lower alkylene group or a carbonyl group, and R⁵ represents a hydrogen atom or group -D-W-R⁶ (wherein D represents group -C(=Z)- (wherein Z represents an oxygen atom or a sulfur atom), group -C(=O)-C(=O)-, or a sulfonyl group; W represents a single bond or group -N(R⁷)- (wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent); and R⁶ represents a hydroxyl group, a lower alkloxy group, a lower alkyl group which may have a substituent, an aryl group which may have a substituent, or a heteroaryl group which may have a substituent);
X represents a single bond, an oxygen atom, a sulfur atom, or a carbonyl group;
Y represents a saturated or unsaturated 5- to 6-membered heterocyclic or cyclohydrocarbon group which may have a substituent, an amino group which may have a substituent, or an aminoalkyl group which may have a substituent; and
the group represented by the following formula: 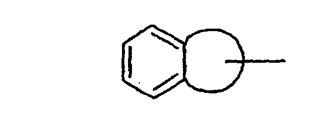 represents a group selected from among an indolyl group, a benzofuranyl group, a benzothienyl group, a benzimidazolyl group, a benzoxazolyl group, a benzothiazolyl group, a naphthyl group, a tetrahydronaphthyl group, and an indanyl group).

Specifically, the substituents represented by R¹ to R⁶ in the above formula (1) include the following.

Examples of the lower alkyl group include C1 to C6 linear, branched, and cyclic alkyl groups. Specific examples of the alkyl groups include methyl, ethyl, propyl, isopropyl, butyl, secondary butyl, tertiary butyl, pentyl, hexyl, cyclopropyl, cyclobutyl, cyclopentyl, and cyclohexyl. The lower alkyl group may have a substituent. Examples of the group which can link to the lower alkyl group include a halogen atom, carboxyl, carbamoyl, amino, cyano, nitro, lower alkanoyl, lower alkoxy, lower alkoxycarbonyl, mono- or di-lower alkylamino, aryl, aralkyloxy, aryloxy, mercapto, lower alkylthio, lower alkylthiocarbonyl, hydroxyl, carbamoyl, and mono- or di-lower alkylaminocarbonyl.

Examples of the lower alkoxy group include C1 to C6 alkoxy groups. Specific examples of the alkoxy groups include methoxy, ethoxy, propoxy, isopropoxy, butoxy, secondary butoxy, and tertiary butoxy.

Examples of the alkoxycarbonyl group include methoxycarbonyl, ethoxycarbonyl, propoxycarbonyl, and butoxycarbonyl.

Examples of the carboxyalkyl group include carboxymethyl, carboxyethyl, and carboxypropyl.

Examples of the alkoxycarbonylalkyl group include methoxycarbonylmethyl, ethoxycarbonylmethyl, propoxycarbonylmethyl, methoxycarbonylethyl, ethoxycarbonylethyl, methoxycarbonylpropyl, and ethoxycarbonylpropyl.

Examples of the carboxyalkoxy group include carboxymethoxy, carboxyethoxy, and carboxypropoxy.

Examples of the alkoxycarbonylalkoxy group include methoxycarbonylmethoxy, ethoxycarbonylmethoxy, propoxycarbonylmethoxy, methoxycarbonylethoxy, and ethoxycarbonylethoxy.

Examples of the hydroxyalkyl group include hydroxymethyl, hydroxyethyl, hydroxypropyl, and hydroxybutyl.

Examples of the C1 to C4 alkylene group include methylene, ethylene, trimethylene, and tetramethylene.

Examples of the mono-lower alkylaminocarbonyl group include methylaminocarbonyl, ethylaminocarbonyl, propylaminocarbonyl, isopropylaminocarbonyl, butylaminocarbonyl, isobutylaminocarbonyl, pentylaminocarbonyl, isopentylaminocarbonyl, hexylaminocarbonyl, and isohexylaminocarbonyl. Examples of the di-lower alkylaminocarbonyl include symmetric dialkylaminocarbonyl groups di-substituted by lower alkyl groups (identical to each other) such as dimetylaminocarbonyl, diethylaminocarbonyl, dipropylaminocarbonyl, diisopropylaminocarbonyl, dibutylaminocarbonyl, and dipentylaminocarbonyl; and asymmetric dialkylaminocarbonyl groups di-substituted by lower alkyl groups (different from each other) such as ethylmethylaminocarbonyl, methylpropylaminocarbonyl, ethylpropylaminocarbonyl, butylmethylaminocarbonyl, butylethylaminocarbonyl, and butylpropylaminocarbonyl.

Examples of the lower alkylsulfonyl group include methylsulfonyl, ethylsulfonyl, propylsulfonyl, isopropylsulfonyl, butylsulfonyl, isobutylsulfonyl, pentylsulfonyl, and isopentylsulfonyl.

Examples of the mono-lower alkylaminothiocarbonyl group include methylaminothiocarbonyl, ethylaminothiocarbonyl, propylaminothiocarbonyl, isopropylaminothiocarbonyl, butylaminothiocarbonyl, isobutylaminothiocarbonyl, pentylaminothiocarbonyl, isopentylaminothiocarbonyl. hexylaminothiocarbonyl, and isohexylaminothiocarbonyl. Examples of the di-loweralkylaminothiocarbonyl include symmetric dialkylaminothiocarbonyl groups di-substituted by lower alkyl groups (identical to each other) such as dimethylaminothiocarbonyl, diethylaminothiocarbonyl, dipropylaminothiocarbonyl, diisopropylaminothiocarbonyl, dibutylaminothiocarbonyl, and dipentylaminothiocarbonyl; and asymmetric dialkylaminothiocarbonyl groups di-substituted by lower alkyl groups (different from each other) such as ethylmethylaminothiocarbonyl, methylpropylaminothiocarbonyl, ethylpropylaminothiocarbonyl, butylmethylaminothiocarbonyl, butylethylaminothiocarbonyl, and butylpropylaminothiocarbonyl.

Examples of the lower alkanoyl group include formyl, acetyl, propionyl, butylyl, isobutylyl, valeryl, isovaleryl, pivaloyl, and hexanoyl. Of these, acetyl, propionyl, and butylyl are preferred, with acetyl and propionyl being more preferred. The lower alkanoyl groups may have a substituent. Examples of the group which can link to the lower alkanoyl group include a halogen atom, carboxyl, carbamoyl, amino, cyano, nitro, lower alkanoyl, lower alkoxy, lower alkoxycarbonyl, mono- or di-lower alkylamino, aryl, aralkyloxy, aryloxy, mercapto, lower alkylthio, lower alkylthiocarbonyl, hydroxyl, carbamoyl, and mono- or di-lower alkylaminocarbonyl.

Examples of the aryl include phenyl, naphthyl, biphenyl, and anthryl. The aryl groups may have a substitutent.

Examples of the heteroaryl group include furyl, thienyl, pyrrolyl, imidazolyl, pyrazolyl, isothiazolyl, isoxazolyl, pyridyl, pyrimidinyl, quinolyl, isoquinolyl, quinazolinyl, quinolidinyl, quinoxalinyl, cinnolinyl, benzimidazolyl, imidazopyridyl, benzofuranyl, naphthylidinyl, 1,2-benzoisoxazolyl, benzooxazolyl, benzothiazolyl, oxazolopyridyl, isothiazolopyridyl, and benzothienyl. Of these, furyl, thienyl, pyrrolyl, imidazolyl, and pyridyl are preferred. The heteroaryl groups may have a substituent. Examples of the group which can link to the aryl group or the heteroaryl group include a halogen atom, carboxyl, amino, cyano, nitro, hydroxyl, lower alkoxy, lower alkoxycarbonyl, mono- or di-lower alkylamino, lower alkanoyl, and a lower alkyl group which may have a substituent.

The substituents represented by Y in formula (1) include the following.

Preferred saturated or unsaturated 5 to 6-membered heterocyclic groups include those having 1 to 2 nitrogen atoms or oxygen atoms serving as hetero atoms. Specific examples of such heterocyclic groups include monovalent heterocyclic groups derived from pyrrolidine, piperidine, imidazoline, piperazine, tetrahydrofuran, hexahydropyrimidine, pyrrole, imidazole, pyrazine, pyrrolidinone, piperidinone, or morpholine. The bonding site of each heterocyclic group is a carbon atom or an nitrogen atom, with a carbon atom bonding site being preferred. These heterocyclic groups may have a substituent, and examples of the substituent include lower alkyl, lower alkanoyl, carbamoyl, monoalkylcarbamoyl, dialkylcarbamoyl, formimidoyl, alkanoimidoyl, benzimidoyl, carboxyl, alkoxycarbonyl, carboxyalkyl, alkylcarbonylalkyl, aminoalkyl, alkanoylamino, alkanoylaminoalkyl, imino, and alkoxycarbonylimino. Examples of more preferred heterocyclic groups include 5 to 6-membered heterocyclic groups represented by the following formula: 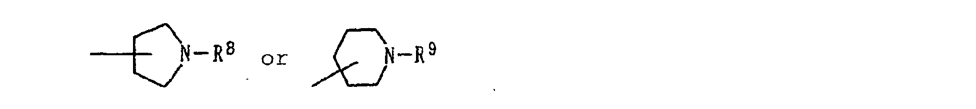 (wherein each of R⁸ and R⁹ represents a hydrogen atom, an amidino group, and a group represented by the formula: 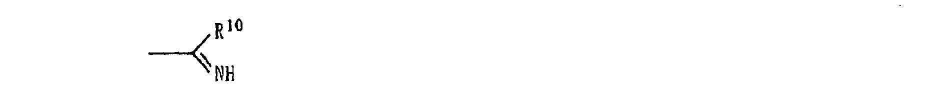 (wherein R¹⁰ represents a lower alkyl group)).

Specific examples the saturated or unsaturated 5- to 6-membered cyclohydrocarbon group include phenyl, cyclohexyl, and cyclopentyl. These cyclohydrocarbon groups may have a substituent, and examples of the substituent include lower alkyl, lower alkanoyl, carbamoyl, monoalkylcarbamoyl, dialkylcarbamoyl, formimidoyl, alkanoimidoyl, benzimidoyl, carboxyl, alkoxycarbonyl, carboxyalkyl, alkylcarbonylalkyl, aminoalkyl, alkanoylamino, alkanoylaminoalkyl, imino, and alkoxycarbonylimino.

Examples of the aminoalkyl group include aminomethyl, aminoethyl, and aminopropyl.

Examples of the group which can link to the amino moiety of the amino group or the aminoalkyl group include a lower alkyl group, a pyrrolidinyl group, a pyrazyl group, a carbamoyl group, a mono-alkylcarbamoyl group, a dialkylcarbamoyl group, a lower alkanoyl group, a formimidoyl group, an alkanoimidoyl group, a benzimidoyl group, and an alkoxycarbonyl group. The aforementioned groups including an alkyl group, an alkoxy group, and an alkanoyl group preferably contains 1 to 6 carbon atoms.

In formula (1), examples of preferred groups represented by the formula: 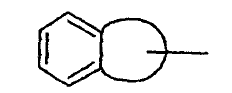 include a benzofuranyl group, a benzimidazolyl group, an indolyl group, a benzothienyl group, a benzothiazolyl group, a naphthyl group, and a tetrahydronaphthyl group.

Among the compounds represented by formula (1), preferred are compounds (including salts thereof) in which each of R¹ to R⁴ is a hydrogen atom; n is 0; X is an oxygen atom; A is a C2 alkylene group or group -CH₂-N(R¹¹)-, which may have one substituent selected from a hydroxyalkyl group, a carboxyl group, an alkoxycarbonyl group, a carboxyalkyl group, and an alkoxycarbonylalkyl group (wherein R¹¹ represents a hydrogen atom or group -SO₂-W¹-R¹² (wherein W¹ represents a single bond or group -N(R¹³)- (wherein R¹³ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent); and R¹² represents a hydroxyl group, a lower alkloxy group, a lower alkyl group which may have a substituent, an aryl group which may have a substituent, or a heteroaryl group which may have a substituent)); Y is a group represented by the following formula: 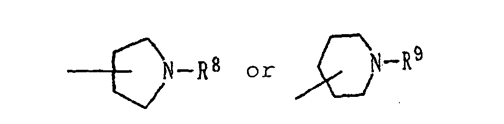 (wherein each of R⁸ and R⁹ represents a hydrogen atom, an amidino group, or a group represented by the formula: 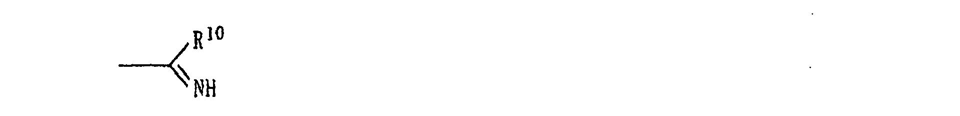 (wherein R¹⁰ represents a lower alkyl group); and the group represented by the formula: 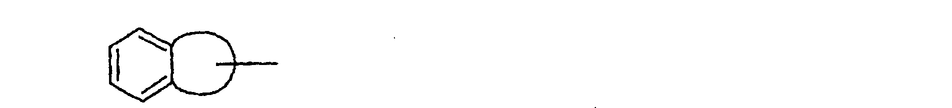 is a benzofuranyl group, a benzimidazolyl group, an indolyl group, a benzothienyl group, a benzothiazolyl group, a naphthyl group, or a tetrahydronaphthyl group).

In some cases, the compounds represented by formula (1) contain an asymmetric carbon atom. In such cases, optical isomers or steroisomers attributable to the asymmetric carbon atom are present. The present invention also encompasses these optical isomers, steroisomers, and mixtures thereof.

No particular limitation is imposed on the salts of the compounds represented by formula (1) so long as the salts are pharmaceutically acceptable. Specific examples include mineral acid salts such as hydrochlorides, hydrobromide, hydroiodides, phosphates, nitrates, and sulfates; organic sulfonic acid salts such as methanesulfonates, 2-hydroxyethanesulfonates, and p-toluenesulfonates; and organic carboxylic acid salts such as acetates, propionates, oxalates, malonates, succinates, glutrates, adipates, tartrates, maleates, malates, and mandelates.

The compound represented by formula (1) or a salt thereof may be present in the non-solvate form or in the form of hydrate or solvate. Thus, the compound of the present invention or a salt thereof encompasses all crystal types thereof and hydrates or solvates thereof.

The aforementioned compounds represented by formula (1), salts thereof, and solvates thereof are known compounds of which production methods and details are disclosed in, for example, Japanese Patent Application Laid-Open (*kokai*) No. 5-208946 and WO 96/16940.

Examples of the aforementioned compounds represented by formula (1) include the following compounds, salts thereof, and solvates thereof:

2-[4-[((3S)-1-acetoimidoyl-3-pyrrolidinyl)oxy]phenyl]-3-(7-amidino-2-naphthyl)propionic acid, (+)-2-[4-[((3S)-1-acetoimidoyl-3-pyrrolidinyl)oxy]phenyl]-3-(7-amidino-2-naphthyl)propionic acid, (2S)-2-[4-[((3S)-1-acetoimidoyl-3-pyrrolidinyl)oxy]phenyl]-3-(7-amidino-2-naphthyl)propionic acid, (2R)-2-[4-[((3R)-1-acetoimidoyl-3-pyrrolidinyl)oxy]phenyl]-3-(7-amidino-2-naphthyl)propionic acid, 2-[4-[(1-acetoimidoyl-4-piperidyl)oxy]phenyl]-3-(7-amidino-2-naphthyl)propionic acid, (+)-2-[4-[(1-acetoimidoyl-4-piperidyl)oxy]phenyl]-3-(7-amidino-2-naphthyl)propionic acid, 2-[4-[(1-acetoimidoyl-4-piperidyl)oxy]phenyl]-3-(5-amidinobenzo[b]thien-2-yl)propionic acid, 2-[4-[((2S)-1-acetoimidoyl-2-pyrrolidinyl)methoxy]phenyl]-3-(5-amidinobenzo[b]thien-2-yl)propionic acid, (+)-2-[4-[((2S)-1-acetoimidoyl-2-pyrrolidinyl)methoxy]phenyl]-3-(5-amidinobenzo[b]thien-2-yl)propionic acid, 3-[4-[((3S)-1-acetoimidoyl-3-pyrrolidinyl)oxy]phenyl]-4-(5-amidinobenzo[b]thien-2-yl)butyric acid, 2-[4-[((3S)-1-acetoimidoyl-3-pyrrolidinyl)oxy]phenyl]-3-(6-amidino-1-ethyl-2-indolyl)propionic acid, 2-[4-[((3R)-1-acetoimidoyl-3-pyrrolidinyl)oxy]phenyl]-3-(6-amidino-1-ethyl-2-indolyl)propionic acid, 2-[4-[(1-acetoimidoyl-9-piperidinyl)oxy]phenyl]-3-(6-amidino-1-ethyl-2-indolyl)propionic acid, N-[4-[(1-acetoimidoyl-4-piperidyl)oxy]phenyl]-N-[(7-amidino-2-naphthyl)methyl]-N'-methylsulfamide, ethyl N-[N-4-[(1-acetoimidoyl-4-piperidyl)oxy]phenyl]-N-[(7-amidino-2-naphthyl)methyl]sulfamoyl]carbamate, 4-[N-4-[(1-acetoimidoyl-4-piperidyl)oxy]phenyl]-N-[(7-amidino-2-naphthyl)methyl]sulfamoyl]benzoic acid, N-[4-[(1-acetoimidoyl-4-piperidyl)oxy]phenyl]-N-[(7-amidino-2-naphthyl)methyl]sulfamoylacetic acid, ethyl N-[N-[4-[(1-acetoimidoyl-4-piperidyl)oxy]phenyl]-N-[(7-amidino-2-naphthyl)methyl]sulfamoyl]glycinate, N-[N-4-[(1-acetoimidoyl-4-piperidyl)oxy]phenyl]-N-[(7-amidino-2-naphthyl)methyl]sulfamoyl]-N-ethoxycarbonylglycine, and N-[N-4-[(1-acetoimidoyl-4-piperidyl)oxy]phenyl]-N-[(7-amidino-2-naphthyl)methyl]sulfamoyl]glycine.

### Examples of preferred compounds represented by (1) include the following:

(2S)-2-[4-[((3S)-1-acetoimidoyl-3-pyrrolidinyl)oxy]phenyl]-3-(7-amidino-2-naphthyl)propionic acid hydrochloride pentahydrate, (+)-2-[4-[(1-acetoimidoyl-4-piperidyl)oxy]phenyl]-3-(7-amidino-2-naphthyl)propionic acid dihydrochrloride, (+)-2-[4-[((2S)-1-acetoimidoyl-2-pyrrolidinyl)methoxy]phenyl]-3-(5-amidinobenzo[b]thien-2-yl)propionic acid dihydrochloride, N-[4-[(1-acetoimidoyl-4-piperidyl)oxy]phenyl]-N-[(7-amidino-2-naphthyl)methyl]sulfamoylacetic acid dihydrochloride, ethyl N-[N-[4-[(1-acetoimidoyl-4-piperidyl)oxy]phenyl]-N-[(7-amidino-2-naphthyl)methyl]sulfamoyl]glycinate dihydrochloride, and N-[N-4-[(1-acetoimidoyl-4-piperidyl)oxy]phenyl]-N-[(7-amidino-2-naphthyl)methyl]sulfamoyl]glycine dihydrochloride.

In addition to the aforementioned compounds represented by formula (1), other examples of the physiologically active substance which may have a substituent and which has an amidino group include the following:

(S)-4-(trans-4-guanidinomethylcyclohexylcarbonylglycyl)-2-oxopiperazine-1,3-diacetic acid, (S)-4-(4-guanidinomethylbenzoylglycyl)-2-oxopiperazine-1,3-diacetic acid, (S)-4-(4-guanidinobenzoylglycyl)-2-oxopiperazine-1,3-diacetic acid, (S)-4-(9-guanidinobenzoylsarcosyl)-2-oxopiperazine-1,3-diacetic acid, (S)-4-(4-guanidinomethylbenzoylsarcosyl)-2-oxopiperazine-1,3-diacetic acid, (S)-1-carboxymethyl-4-(4-guanidinobenzoylsarcosyl)-2-oxopiperazine-3-propionic acid, (S)-4-(3-guanidinophenylacetylglycyl)-2-oxopiperazine-1,3-diacetic acid, (S)-4-(4-amidinobenzoylglycyl)-2-oxopiperazine-1,3-diacetic acid, 4-(4-amidinobenzoylglycyl)-2-oxopiperazine-1-acetic acid, (S)-4-(4-amidinobenzoylglycyl)-3-methoxycarbonylmethyl-2-oxopiperazine-1-acetic acid, (S)-4-(4-amidinobenzoylglycyl)-3-benzyl-2-oxopiperazine-1-acetic acid, (S)-4-(4-amidinobenzoylglycyl)-3-carbamoylmethyl-2-oxopiperazine-1-acetic acid, (S)-4-(4-amidinobenzoylglycyl)-1-carboxymethyl-2-oxopiperazine-3-propionic acid, 4-(4-amidinobenzoyl)aminoacetyl-3-[3-(4-amidinobenzoyl)aminopropyl]-2-oxopiperazine-1-acetic acid, 4-(4-amidinobenzoyl)aminoacetyl-3-[4-(4-amidinobenzoyl)aminobutyl]-2-oxopiperazine-1-acetic acid, 4-(4-amidinobenzoyl)aminoacetyl-3-[2-(4-amidinobenzoyl)aminoethyl]-2-oxopiperazine-1-acetic acid, 4-(4-amidinobenzoyl)aminoacetyl-3-[2-(4-amidinophenylaminocarbonyl)ethyl]-2-oxopiperazine-1-acetic acid, 4-(4-amidinobenzoyl)aminoacetyl-3-[3-(4-amidinophenylaminocarbonyl)propyl]-2-oxopiperazine-1-acetic acid, 4-(4-amidinobenzoyl)aminoacetyl-3-[4-(4-amidinophenylaminocarbonyl)butyl]-2-oxopiperazine-1-acetic acid, (S)-4-(4-guanidinobenzoylamino)acetyl-3-[3-(4-guanidinobenzoylamino)propyl]-2-oxopiperazine-1-acetic acid, (S)-4-(4-amidinobenzoylamino)acetyl-3-[2-(4-guanidinobenzoylamino)ethyl]-2-oxopiperazine-1-acetic acid, (S)-4-[4-(2-aminoethyl)benzoylamino]acetyl-3-[3-(4-amidinobenzoylamino)]propyl-2-oxopiperazine-1-acetic acid, (S,S)-3-3-(4-guanidinobenzoylamino)propyl]-4-[3-(4-methoxyphenyl)-2-[4-(5-trifluoromethyl-[1,2,4]oxadiazol-3-yl-amino)benzoylamino]propionyl]-2-oxopiperazine-1-acetic acid, and (S,S)-4-[2-(4-guanidinobenzoyl)amino-3-(4-methoxyphenyl)propionyl]-3-[3-(4-guanidinobenzoyl)aminopropyl]-2-oxopiperazine-1-acetic acid.

The aforementioned compounds may be in the form of pharmaceutically acceptable ester, salt, or solvate. These compounds may be optical isomers or racemic modifications. The aforementioned compounds, salts thereof, and solvates thereof are known compounds of which production methods and details are disclosed in, for example, Japanese Patent Application Laid-Open (*kokai*) No. 6-25285.

The aqueous solution of the present invention may be produced by dissolving a physiologically active substance which may have an appropriate amount of a substituent and which has an amidino group and an optional appropriate additive in an appropriate amount in an appropriate aqueous solvent and adjusting the pH of the solution by use of an appropriate pH adjusting agent to a desired value. Examples of the aqueous solvent include water for injection, physiological saline, Ringer's solution, ethanol, propylene glycol, and polyethylene glycol. Examples of the additive include a stabilizing agent, a solubilizing agent, a buffer, a tonicity agent, and a soothing agent. These additives may be incorporated into the aqueous solution in such an amount that the effects of the present invention are not impaired. Examples of the pH adjusting agent include acids and alkaline substances which are not harmful to the human bodies, etc. Specific examples include inorganic acids such as hydrochloric acid and sulfuric acid; inorganic bases such as sodium hydroxide; organic acids such as lactic acid and citric acid; and organic bases such as meglumine and monoethanolamine. According to the present invention, the aqueous solution containing a physiologically active substance which may have a substituent and which has an amidino group is required to have a pH higher than 2 and equal to or lower than 4 from the viewpoint of drug preparation's stability. The pH is preferably higher than 2 and lower than 4, more preferably higher than 2 and equal to or lower than 3.5, most preferably 2.5 to 3.5.

The concentration of the physiologically active substance which may have a substituent and which has an amidino group in the aqueous solution may be appropriately adjusted in accordance with the activity (pharmacological effect), and is generally 0.01 to 500 mg/mL, preferably 0.1 to 300 mg/mL, more preferably 1 to 100 mg/mL.

The lyophilized product of the present invention may be produced through lyophilization of the aforementioned aqueous solution at a temperature equal to or lower than the eutectic point. Generally, since the eutectic point falls in a multicomponent system, lyophilization must be performed under low temperature conditions, which are disadvantageous in industrial production. Therefore, a small number of components is advantageous for producing a lyophilized product. In this connection, preferred is a product obtained by lyophilizing an aqueous solution containing a physiologically active substance which may have a substituent and which has an amidino group and a pH adjusting agent serving as solutes.

The aqueous solution and the lyophilized product of the present invention may serve as an injection for human bodies, etc. Thus, the aqueous solution and the lyophilized product are preferably sterilized. For sterilization, the aqueous. solution is sterilized through a method; e.g., sterilization by autoclave or sterilization through filtration by use of a membrane filter (pore size: 0.22 µm) or a similar filter. A sterilized lyophilized product can be produced by lyophilizing a sterilized aqueous solution.

The thus-produced lyophilized product is dissolved in a solvent upon use (e.g., water for injection or buffer), and serves as an injection. When the solution having a pH lower than 4 is administered to a human body, etc., local irritation may be induced. Thus, the pH of the solution after dissolution is preferably adjusted to 4 or higher by regulating the pH of the solvent for reconstitution prior to use or diluting the solution for elevating pH. The solution prepared by dissolving the lyophilized product of the present invention in a solvent is remarkably stable. The aforementioned aqueous solution or lyophilized product may be combined with a solvent upon use, thereby providing an injection kit.

### Examples

The present invention will next be described in detail by way of examples, which should not be construed as limiting the invention thereto.

### Comparative Example 1

(2S)-2-[4-[((3S)-1-Acetoimidoyl-3-pyrrolidinyl)oxy]phenyl]-3-(7-amidino-2-naphthyl)propionic acid hydrochloride pentahydrate (hereinafter abbreviated as compound A) (963.75 mg) was weighed and dissolved in water for injection (about 40 mL). Water for injection was further added to the solution so as to adjust total volume to 50 mL. The pH of the solution was found to be 6.6. The solution was filtered through a membrane filter (pore size: 0.22 µm), and the filtrate was filled into each 10-mL amber vial (2.0 mL/vial). The filled solution was lyophilized by means of a freeze dryer, and the vials were capper with a rubber stopper and sealed with aluminum caps, to thereby produce comparative formulation samples. The total impurity amount contained in each of the thus-prepared lyophilized preparations was determined through HPLC at the start of storage and after 2-week storage at 60°C. Table 1 shows the formulation of the drug preparation per vial and the total impurity amount (%) of the preparation determined at the start of storage and after 2-week storage at 60°C.

**Table 1**

| Component | Comparative formulation | |
|---|---|---|
| Compound A (Water for injection) | | 19.275 mg (Total 2 mL) |
| Total impurity mass (%) | Start of storage | 0.31 |
| | 60°C 2 weeks after | 5.21 |

As is clear from Table 1, the total impurity amount of the drug preparation of the comparative formulation after 2-week storage at 60°C was increased to about 5%, indicating that the comparative formulation was unstable.

### Example 1

Compound A (963.75 mg) was weighed and dissolved in water for injection (about 40 mL), and a predetermined volume of 0.1N hydrochloric acid was added to the solution. Water for injection was further added to the solution so as to adjust total volume to 50 mL. The pH values of the solution samples were adjusted to 2.5, 3.0, 3.5, 4.0, and 5.0, respectively. Each solution sample was filtered through a membrane filter (pore size: 0.22 µm), and the filtrate was filled into each 10-mL amber vial (2.0 mL/vial). The vials containing the liquid were subjected to lyophilization by means of a lyophilization apparatus, and the vials were closed by use of a rubber stopper and sealed with an aluminum cap, to thereby produce drug preparations of specific pH values. The formulation of a drug preparation produced from an aqueous solution having a pH of 2.5 is represented by formulation 1, and similarly, formulations 2, 3, 4, and 5 correspond to pH values of 3.0, 3.5, 4.0, and 5.0, respectively. Through observation the vials, all these lyophilized drug preparations were confirmed to form dry cake. The total impurity amount contained in each of the thus-prepared lyophilized preparations was determined through HPLC at the start of storage and after 2-week storage at 60°C. Table 2 shows the formulation of each pH drug preparation per vial and the total impurity amount (%) of the preparation determined at the start of storage and after 2-week storage at 60°C.

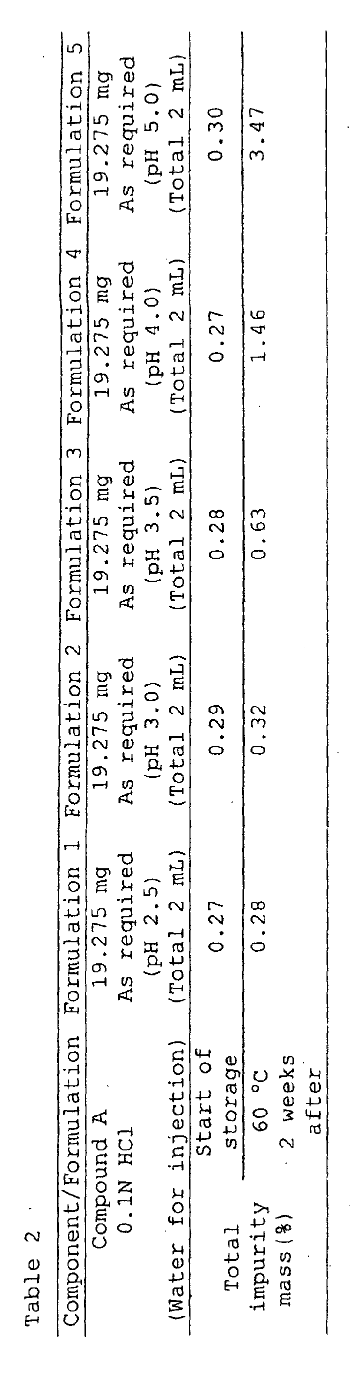

As is clear from Table 2, the total impurity amount of the drug preparations after 2-week storage at 60°C was reduced as the pH was lowered. In particularly, drug preparations produced from an aqueous solution having a pH of 3.0 or lower (formulations 1 and 2) exhibited virtually no increase in total impurity amount (%) after 2-week storage at 60°C.

Accordingly, as compared with the comparative formulation (without adjusting pH), drug preparations of formulations 1 to 4 (pH≤4.0) were confirmed to be remarkably stable.

Since the lyophilized product of the present invention assumes the form of dry cake, error of product inspection caused by failure to form dry cake can be prevented. In addition, drug preparations can be produced from only a small number of ingredients, and thus raw material cost can be suppressed, and the time for lyophilization can be shortened. As a result, cost for industrial production can be reduced.

### Industrial applicability

The aqueous solution of the present invention serves as an excellent starting material for producing a lyophilized product which can be stored for a long period of time. The lyophilized product produced by lyophilizing the aqueous solution assumes the form of dry cake and has improved stability during long-term storage.

Therefore, the present invention advantageously enables long-term storage of a physiologically active substance which may have a substituent and which has an amidino group.

## Claims

1. An aqueous solution containing a physiologically active substance which may have a substituent and which has an amidino group, the solution having a pH higher than 2 and equal to or lower than 4.

2. An aqueous solution containing a physiologically active substance which may have a substituent and which has an amidino group, the solution having a pH higher than 2 and lower than 4.

3. An aqueous solution containing a physiologically active substance which may have a substituent and which has an amidino group, the solution having a pH higher than 2 and equal to or lower than 3.5.

4. An aqueous solution containing a physiologically active substance which may have a substituent and which has an amidino group, the solution having a pH of 2.5 to 3.5.

5. A lyophilized product produced by lyophilizing an aqueous solution containing a physiologically active substance which may have a substituent and which has an amidino group, the solution having a pH higher than 2 and equal to or lower than 4.

6. A lyophilized product produced by lyophilizing an aqueous solution containing a physiologically active substance which may have a substituent and which has an amidino group, the solution having a pH higher than 2 and lower than 4.

7. A lyophilized product produced by lyophilizing an aqueous solution containing a physiologically active substance which may have a substituent and which has an amidino group, the solution having a pH higher than 2 and equal to or lower than 3.5.

8. A lyophilized product produced by lyophilizing an aqueous solution containing a physiologically active substance which may have a substituent and which has an amidino group, the solution having a pH of 2.5 to 3.5.

9. An aqueous solution as described in any one of claims 1 to 4 or a lyophilized product as described in any one of claims 5 to 8, wherein the physiologically active substance which may have a substituent and which has an amidino group is a compound represented by the formula (1): 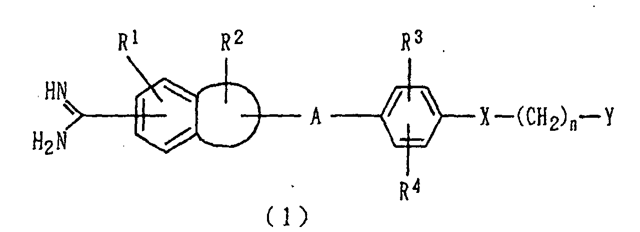 (wherein R¹ represents a hydrogen atom or a lower alkoxy group;
R² represents a hydrogen atom, a lower alkyl group, a lower alkoxy group, a carboxyl group, an alkoxycarbonyl group, a carboxylalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxy group, or an alkoxycarbonylalkoxy group;
R⁴ represents a hydrogen atom, a halogen atom, an amino group, a cyano group, a nitro group, a hydroxyl group, a lower alkyl group, or a lower alkoxy group;
n is a number from 0 to 4;
A represents a C1 to C4 alkylene group or group -B-N(R⁵)-, which may have 1 to 2 substituents selected from among a hydroxyalkyl group, a carboxyl group, an alkoxycarbonyl group, a carboxyalkyl group, and an alkoxycarbonylalkyl group (wherein B represents a lower alkylene group or a carbonyl group, and R⁵ represents a hydrogen atom or group -D-W-R⁶ (wherein D represents group -C(=Z)- (wherein Z represents an oxygen atom or a sulfur atom), group -C(=O)-C(=O)-, or a sulfonyl group; W represents a single bond or group -N(R⁷)- (wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent); and R⁶ represents a hydroxyl group, a lower alkloxy group, a lower alkyl group which may have a substituent, an aryl group which may have a substituent, or a heteroaryl group which may have a substituent);
X represents a single bond, an oxygen atom, a sulfur atom, or a carbonyl group;
Y represents a saturated or unsaturated 5- to 6-membered heterocyclic or cyclohydrocarbon group which may have a substituent, an amino group which may have a substituent, or an aminoalkyl group which may have a substituent; and
the group represented by the following formula: 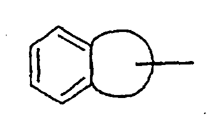 represents a group selected from among an indolyl group, a benzofuranyl group, a benzothienyl group, a benzimidazolyl group, a benzoxazolyl group, a benzothiazolyl group, a naphthyl group, a tetrahydronaphthyl group, and an indanyl group), a salt thereof, or a solvate of the compound or salt.

10. An aqueous solution as described in any one of claims 1 to 4 or a lyophilized product as described in any one of claims 5 to 8, wherein the physiologically active substance which may have a substituent and which has an amidino group is one compound or two or more compounds in combination selected from the following compounds:
2-[4-[((3S)-1-acetoimidoyl-3-pyrrolidinyl)oxy]phenyl]-3-(7-amidino-2-naphthyl)propionic acid, (+)-2-[4-[((3S)-1-acetoimidoyl-3-pyrrolidinyl)oxy]phenyl]-3-(7-amidino-2-naphthyl)propionic acid, (2S)-2-[4-[((3S)-1-acetoimidoyl-3-pyrrolidinyl)oxy]phenyl]-3-(7-amidino-2-naphthyl)propionic acid, (2R)-2-[4-[((3R)-1-acetoimidoyl-3-pyrrolidinyl)oxy]phenyl]-3-(7-amidino-2-naphthyl)propionic acid, 2-[4-[(1-acetoimidoyl-4-piperidyl)oxy]phenyl]-3-(7-amidino-2-naphthyl)propionic acid, (+)-2-[4-[(1-acetoimidoyl-4-piperidyl)oxy]phenyl]-3-(7-amidino-2-naphthyl)propionic acid, 2-[4-[(1-acetoimidoyl-4-piperidyl)oxy]phenyl]-3-(5-amidinobenzo[b]thien-2-yl)propionic acid, 2-[4-[((2S)-1-acetoimidoyl-2-pyrrolidinyl)methoxy]phenyl]-3-(5-amidinobenzo[b]thien-2-yl)propionic acid, (+)-2-[4-[((2S)-1-acetoimidoyl-2-pyrrolidinyl)methoxy]phenyl]-3-(5-amidinobenzo[b]thien-2-yl)propionic acid, 3-[4-[((3S)-1-acetoimidoyl-3-pyrrolidinyl)oxy]phenyl]-4-(5-amidinobenzo[b]thien-2-yl)butyric acid, 2-[4-[((3S)-1-acetoimidoyl-3-pyrrolidinyl)oxy]phenyl]-3-(6-amidino-1-ethyl-2-indolyl)propionic acid, 2-[4-[((3R)-1-acetoimidoyl-3-pyrrolidinyl)oxy]phenyl]-3-(6-amidino-1-ethyl-2-indolyl)propionic acid, 2-[4-[(2-acetoimidoyl-4-piperidinyl)oxy)phenyl]-3-(6-amidino-1-ethyl-2-indolyl)propionic acid, N-[4-[(1-acetoimidoyl-4-piperidyl)oxy]phenyl]-N-[(7-amidino-2-naphthyl)methyl]-N'-methylsulfamide, ethyl N-[N-4-[(1-acetoimidoyl-4-piperidyl)oxy]phenyl]-N-[(7-amidino-2-naphthyl)methyl]sulfamoyl]carbamate, 4-[N-4-[(1-acetoimidoyl-4-piperidyl)oxy]phenyl]-N-[(7-amidino-2-naphthyl)methyl]sulfamoyl]benzoic acid, N-[4-[(1-acetoimidoyl-4-piperidyl)oxy]phenyl]-N-[(7-amidino-2-naphthyl)methyl]sulfamoylacetic acid, ethyl N-[N-[4-[(1-acetoimidoyl-4-piperidyl)oxy]phenyl]-N-[(7-amidino-2-naphthyl)methyl]sulfamoyl]glycinate, N-[N-4-[(1-acetoimidoyl-4-piperidyl)oxy]phenyl]-N-[(7-amidino-2-naphthyl)methyl]sulfamoyl]-N-ethoxycarbonylglycine, and N-[N-4-[(1-acetoimidoyl-4-piperidyl)oxy]phenyl]-N-[(7-amidino-2-naphthyl)methyl]sulfamoyl]glycine, a salt thereof, and a solvate of the compound or salt, a salt thereof, and a solvate of the compound or salt.

11. An aqueous solution as described in any one of claims 1 to 4 or a lyophilized product as described in any one of claims 5 to 8, wherein the physiologically active substance which may have a substituent and which has an amidino group is (2S)-2-[4-[((3S)-2-acetoimidoyl-3-pyrrolidinyl)oxy]phenyl]-3-(7-amidino-2-naphthyl)propionic acid hydrochloride pentahydrate.

12. An injection comprising an aqueous solution as recited in any one of claims 1 to 4 and 9 to 11 or a lyophilized produced as recited in any one of claims 5 to 11.

13. An injection kit comprising a solvent for reconstruction prior to use and an injection as recited in claim 12.
